# EUROPEAN PATENT APPLICATION

(11) **EP 4 591 836 A1**
(43) Date of publication of application: **30.07.2025**
(21) Application number: 24154299.2
(22) Date of filing: 28.01.2024
(51) Int. Cl.: A61F 2/78, A61F 2/80, A61F 2/50

(54) **MOOR FLEXIFORM**

(71) Applicant: Moor Ortotika in Protetika d.o.o., 8261 Jesenice na Dolenjskem (SI)
(72) Inventor: BOSTJAN, Boltezar, 1000 Ljubljana (SI); ERZAR, Dominik, 1310 Ribnica (SK)

(57) **Abstract**

The significance of the prosthetic socket in determining the overall satisfaction with a prosthesis is highlighted, emphasizing its role in either enhancing or diminishing user experience. A well-fitted socket is crucial to avoid prosthesis abandonment and encourage active community engagement. The product focuses on the advantages of adjustable socket designs, noting their ability to adapt to changes in residual limb volume and reduce socket pressure, thereby enhancing the fit and comfort of the prosthesis for the user.

## Description

### Technical field to which invention relates

The present invention relates to a premium prosthetic socket offering dynamic flexibility, skin-friendly comfort, and precise volume management.

### Background of the invention

Adjustable sockets, while not a novel invention, have seen a surge in popularity, evidenced by an uptick in related publications, patents, and the availability of diverse designs in the market. In comparing these sockets, it's essential to understand the key principles that guide their adjustability. These include inflatable bladders, movable panels, circumferential adjustment, and variable length. Additionally, their surface forms can be categorized into three types: conformable, rigid with a single degree of freedom (DOF), and rigid with multiple DOFs.

When comparing commercially available adjustable sockets to those in research phases, a distinct contrast emerges. Commercial products often rely on panels or circumferential adjustments for their adaptability, whereas research prototypes are gravitating towards bladder-based systems. Yet, across both commercial and research domains, there's a notable gap in reporting the rationale behind specific adjustment locations and ranges.

In terms of minimal volume limitation, certain adjustable socket designs have inherent constraints. Notably, straps and pumps are the only actuation mechanisms that incorporate safety features to prevent over or under-tightening, thereby reducing the risk of tissue damage or socket detachment. However, safety considerations are frequently overlooked in the design and description of these sockets.

Looking at the context of low-resource settings, off-the-shelf adjustable sockets hold significant promise. They offer a practical solution, requiring only minor modifications by clinicians for a proper fit and allowing end-users to make small adjustments. This approach could drastically reduce the need for frequent clinic visits and fitting sessions. Despite their potential, only a few designs specifically address the needs of low-resource environments, and research in these settings remains limited.

In comparing these sockets, it's also important to acknowledge the limitations in the existing literature. Most studies are available only in English or French and involve small participant groups, limiting the scope of real-world testing for adjustable sockets. Additionally, the trade-offs associated with these sockets, such as higher costs and increased complexity, are often underreported.

Future comparisons should aim to close the gap between industry and academic research. A significant number of commercial designs lack empirical backing from clinical studies. Applying scientific methods to evaluate these designs could shed light on their effectiveness and suitability for different patient groups. Another area ripe for exploration is the definition of a well-fitting prosthetic socket. Investigating the interplay between socket design, mechanical coupling, comfort, and the health of residual limb tissue could lead to significant advancements in socket technology. Adjustable sockets, due to their inherent versatility, could play a crucial role in patient satisfaction.

### Technical problem to be solved

The objective of the present invention is to provide an individually made prosthetic socket to increase comfort, stability and satisfaction with a prosthetic device of a user.

### Disclosure of invention

MOOR FlexiForm is a premium prosthetic socket offering dynamic flexibility, skin-friendly comfort, and precise volume management. It is available for Transtibial, Knee Exarticulation, and Transfemoral amputees, making it a versatile choice for various amputation levels.

Designed with the user's lifestyle in mind, it features an integrated comfort system, aesthetic excellence, non-stick finish, and cosmetic compatibility. Each socket is uniquely tailored for an ideal fit and stylish functionality, which increase comfort, stability and satisfaction with a prosthetic device of a user.

### Brief description of the drawings

FIGURE 1, 2, 3, 4 show different possible locations of compression pads. Compression system can be used in all variety of prosthetic sockets
FIGURE 1: show 3 point pressure system in TT socket
FIGURE 2: show 2 point pressure system in TT socket
FIGURE 3: show 4 point pressure system in IC TF socket
FIGURE 4: show 3 point pressure system in QL TF socket
FIGURE 5: show 1 point pressure system in IC TF socket
FIGURE 6: show 2 point pressure system in QL TF socket
FIGURE 7: shows transversal cut of IC TF socket
FIGURE 8: shows transversal cut of TT socket
FIGURE 9: shows integrated carbon stabilizing frame in TT socket
FIGURE 10: shows sagital side of position of air pressure pads

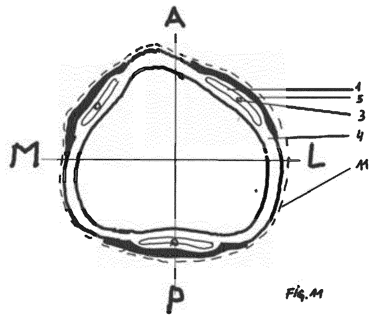

FIGURE 11: shows transversal cut of all 3 layers of the TT socket

### Detailed description of the drawings

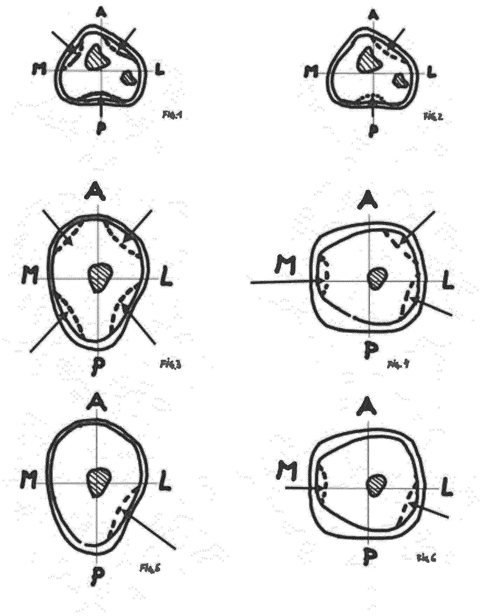

The Moor FlexiForm socket system is innovatively designed to utilize compression of soft tissue within a variety of prosthetic socket types and levels. This system allows for adjustable compression, customizable in size, location, and the number of compression pads. Figure 1 exemplifies this with a three-point compression arrangement, one among numerous potential configurations.

Each pad within the system is modular, offering the capability for alteration, repositioning, resizing, and personalization to suit any socket shape and design, as depicted in Figures 2 through 6.

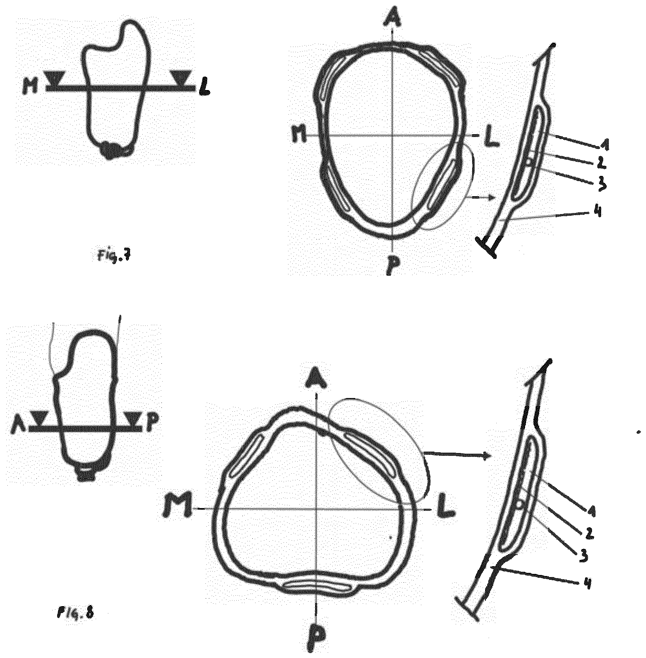

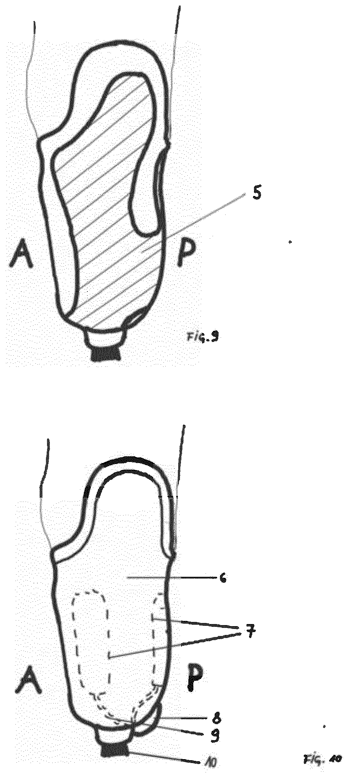

The prosthetic socket comprises four distinct layers, each serving a specific purpose. As depicted in Figure 7, the first layer is a transverse section of the socket, consisting of a rigid High-Temperature Vulcanized (HTV) silicone, denoted as number 4, integrated with an air pressure pad marked as number 1. This pad includes a reinforcement layer indicated as number 2 and an air tube connection labeled as number 3. A similar principle is applied to the TT socket, as described in Figure 8. The pads are integrated and connected to a collection tube valve located at the distal end of the socket, demonstrated in Figure 10.

The second layer comprises a rigid carbon fiber frame, illustrated in Figure 9 as item number 5. This carbon fiber framework is essential and is customized based on the patient's requirements, whether for TT, AK or KX sockets.

The third layer consists of a soft HTV silicone cover. Connection for the airways, designated as number 9, is established at the distal end of the socket and is integrated with a manual air pump, marked as number 10.

The fourth and final layer is a finishing layer, purposed for a non-stick exterior. The culmination of these components results in a flexible socket characterized by an internal carbon fiber stabilization core and an integrated volume management pneumatic system.

## Claims

1. Adaptive Flexibility: Constructed from premium medical-grade HTV silicon, MOOR-FlexiForm is designed for dynamic flexibility, adapting to every movement with ease.

2. Skin-Friendly Material: MOOR-FlexiForm is crafted with a focus on skin health, featuring softly contoured edges and a soft silicone edge that prevents skin abrasion. Coupled with its skin-compatible material, this design ensures all-day comfort without irritation, providing a gentle interface with the user's body.

3. Universal Suspension Compatibility: MOOR-FlexiForm is compatible with all suspension methods, including vacuum, pin, lanyard, and kiss / summit lock system, offering versatility in usage.

4. Volume Management: System not only enables users to precisely adjust the socket's fit in response to changes in stump volume, ensuring an ideal and consistent fit throughout the day, but also offers the convenience of adjusting the fit without the need to undress. This innovative feature allows for adjustment over clothing, providing ease and discretion in managing the fit of the prosthetic socket.

5. Integrated Comfort System: The built-in pneumatic pad, along with an external pneumatic pump, enables micro-adjustments, providing a tailored fit unique to user's needs.

6. Aesthetic: MOOR-FlexiForm not only boasts a design that's as stylish as it is functional, but it is also clothing-friendly, ensuring it does not damage or snag on clothing fabrics.

7. Non-Stick Finish: The socket's unique non-stick slippery outer finish guarantees smooth movement against clothing, enhancing comfort and mobility.

8. Bespoke Tailoring: Each MOOR-FlexiForm is custom-made to the user's exact specific measurements, ensuring a socket that feels like a natural extension of their body.

9. Cosmesis-Friendly: MOOR-FlexiForm is compatible with prosthetic cosmesis, allowing for aesthetic personalization without compromising functionality.

10. Sitting Comfort: Designed with an emphasis on comfort, especially while sitting, it provides an experience for the user.

11. Enhanced Range of Motion: For transfemoral amputees, MOOR-FlexiForm allows improved hip motion, and for transtibial, it enhances knee mobility, promoting a more natural movement.
